# EUROPEAN PATENT SPECIFICATION

(11) **EP 0 678 300 B1**
(45) Date of publication and mention of the grant of the patent: **19.04.2000**
(21) Application number: 95900273.4
(22) Date of filing: 07.11.1994
(51) Int. Cl.: A61L 27/00

(54) **BONE SUBSTITUTE MATERIAL AND PROCESS FOR PRODUCING THE SAME**
KNOCHENERSATZMATERIAL UND VERFAHREN ZU SEINER HERSTELLUNG
MATERIAU DE SUBSTITUTION OSSEUSE ET PROCEDE DE PRODUCTION DUDIT MATERIAU

(30) Priority: 09.11.1993 JP 30465993
(43) Date of publication of application: 25.10.1995
(73) Proprietor: THE FOUNDATION FOR THE PROMOTION OF ION ENGINEERING, Kyoto-shi, Kyoto 606 (JP)
(72) Inventor: KOKUBO, Tadashi, Kyoto 617 (JP)
(74) Representative: Jung, Elisabeth, Dr.
(86) International application number: PCT/JP94/01885
(87) International publication number: WO 95/13100

(56) References cited:
- EP-A- 0 264 354
- FR-A- 2 318 617
- JP-A- 3 073 157
- JP-A- 5 057 010
- THIN SOLID FILMS, vol. 227, no. 1, 5 May 1993 pages 32-36, XP 000367364 TORRISI L ET AL 'THERMALLY ASSISTED HYDROXYAPATITE OBTAINED BY PULSED-LASER DEPOSITION ON TITANIUM SUBSTRATES'

## Description

### Technical Field

This invention relates to a material suitable for use as a bone substitute and the manufacturing method thereof. This material for a bone substitute may be utilized effectively as a preparing material for parts where a large load is applied, such as femoral bones, hip joints and tooth roots.

### Background Art

The criterion considered for an artificial material to exhibit bioactivity, i.e., an ability to bond with bones in the body, is the material's capability of forming an apatite layer, which is of the same type as the inorganic components in bone, on their surface.

Conventionally employed materials for a bone substitutes which exhibit such a characteristic are: Na₂O-CaO-SiO₂-P₂O₅ glass, sintered hydroxyapatite (Ca₁₀(PO₄)₆(OH)₂), and MgO-CaO-SiO₂-P₂O₅ glass-ceramic. These are superior materials with a bioactivity, a characteristic to form an apatite layer similar to the inorganic components in bone on their surface in the body, and to bond with bones directly. However, their values of fracture toughness (1 to 2 MPam^{1/2}) are not nearly comparative to that of a human cortical bone (2 to 6 MPam^{1/2}), therefore they may not be utilized as a material for substituting in the hip joints and tibial bones where a large load is applied.

Therefore, the substitute materials currently utilized in these locations are titanium, which exhibits the most superior biocompatibility among the metallic materials, and its alloys. These metallic materials possess a large value of fracture toughness, however they take a long time, about ten years, to directly bond with bones.

Therefore, a bioactivity has been provided to these metallic materials, by the method of plasma coating, to cover their surface with a molten hydroxyapatite. The materials obtained by this method offer both the fracture toughness of titanium and the bioactivity of apatite.

However, there are the following problems in the preparation method by the plasma coating: (1) necessity of an expensive plasma spray instrument, (2) difficulty in controlling the composition and the crystallinity of the hydroxyapatite formed on the metallic substrate surface since the hydroxyapatite powder, the sprayed material, is instantaneously but once exposed to about 30,000 °C of high temperature, (3) difficulty in forming a dense apatite layer since the method only sediments the half-molten hydroxyapatite powder on the substrate by free fall, and (4) difficulty in forming a strong bond of the apatite layer to the substrate due to the same reason as above.

### Disclosure of Invention

It is an object of the present invention to solve these conventional problems and to offer the material for a bone substitute, which provides both the fracture toughness of titanium and the bioactivity of apatite, wherein titanium and apatite are tightly adhered, and which is available at low cost.

To achieve the above object, the material for a bone substitute of the present invention comprises a substrate made of titanium (Ti) or titanium (Ti) alloys and a surface layer (a primary surface layer) formed on the surface of the substrate, said primary surface layer comprising a titanium oxide phase and amorphous phases of alkali titanates. As a substrate, pure Ti is desirable from the view point of biocompatibility, while alloys thereof such as Ti-6Al-4V, Ti-5Al-2.5Sn, Ti-3Al-13V-11Cr, Ti-15Mo-5Nb-3Ta, Ti-6Al-2Mo-Ta are preferred from the forming view point.

In addition, the material is optionally provided with a second layer, of which the main constituent is apatite, further on top of the primary surface layer.

The desirable type of the primary surface layer has a gradually decreasing concentration of the titanium oxide phase towards the outer surface, along with a gradually increasing concentration of the total alkali ions towards the outer surface. The desirable thickness of the primary surface layer is about 0.1 to 10 µm, preferably about 1 µm, while that of the second layer is at least 1 µm, preferably about 10 µm.

The suitable production method to prepare the said materials for a bone substitute comprises soaking the substrate made of titanium (Ti) or titanium (Ti) alloys into alkaline solution, followed by heating the resultant substrate to the level of at most the titanium (Ti) or titanium alloys transition temperature. In addition, after the heat treatment, the substrate may be soaked into the aqueous solution which contains calcium (Ca) and phosphorous (P) to a level of, at least, the apatite solubility, such as the simulated body fluid.

Here, the alkaline solution is desirably an aqueous solution which contains at least one of the following ions: sodium ions Na+, potassium ions K⁺, and calcium ions Ca²⁺. The heating temperature is desirable from 300 to 800 °C, and more preferably from 550 to 650 °C.

Originally there exists an extremely thin film on the surface of titanium (Ti) or titanium (Ti) alloys, which comprises oxides similar TiO₂. TiO₂ is an amphoteric substance which reacts with both strong acids and bases. Therefore, soaking of the substrate made of titanium (Ti) or titanium alloys into alkaline solution results in the amorphous alkali titanates on the substrate surface with a concentration gradient gradually increasing from the inside where very little reaction takes place, to the outside where more reaction occurs. Then the substrate is heated to the level of at most the titanium (Ti) or titanium alloys transition temperature, which causes the diffusion of oxygen and increases the thickness of the said formed phase.

By this method, the surface layer consisting essentially of the titanium oxide phase and the amorphous alkali titanate phase is formed on the surface of the substrate. In addition, the alkali titanate produced by the intermediate process exhibits a gradual concentration gradient, wherein the alkali titanate concentration gradually increases toward the outside along the surface thickness direction. Therefore the titanium oxide phase, the starting material of the said compound, gradually decreases toward the outside. On the other hand, the total concentration of alkali ions (Na⁺, K⁺, and Ca²⁺ and so on) consisted in the product component, an amorphous alkali titanate phase, increases gradually toward the outside. The slopes of these concentration changes are so gradual that the interface between the substrate and the surface layer and the boundaries among the phases within the surface layer are tightly bonded. This is the major difference from the case when substrate is soaked into a preliminarily prepared titania gel in order to form a gel which easily bonds to bones on the Ti surface, but because the adhesive strength between the Ti and the gel layer is weak, the layer can be peeled off. In the present invention the outer surface is rich in alkali ions, which can be exchanged with hydrogen ions within a simulated body fluid or a actual body fluid, to form a titanium hydroxide phase which readily reacts with calcium (Ca) and phosphorous (P).

On the other hand, the aqueous solutions or body fluids which consist of calcium (Ca) and phosphorous (P) at a level at least of the apatite's solubility comprise the components which grow apatite. Therefore they are capable of growing apatite crystals. However, in reality, the activation energy for the nucleation of the apatite is so high that this barrier prevents spontaneous nucleation of the apatite in the solution or body fluid. On the other hand, the amorphous titanium hydroxide phase formed by the hydration of alkali titanate phase is highly reactive owing to its amorphous structure. Therefore it forms apatite nuclei by reacting with the bone forming components in the body fluid.

Further, apatite nuclei may be preliminarily formed by being soaked into the aqueous solution, more desirably the simulated body fluids, which consist of calcium (Ca) and phosphorous (P) at a level of at least the apatite's solubility, after the heat treatment. Those particularly treated by the simulated body fluid which possesses the ion concentration close to that of the actual body fluid easily bond with bones, since the composition and the structure of the apatite formed on the surface is very similar to those of bones.

Here, the heat treatment temperature less than 300 °C will not permit diffusion of air into the material at a satisfactory rate, thereby leading to an insufficiency of oxygen supply in the surface layer. As a result, the primary surface layer will not be thick enough and its ability to form apatite on its surface in the body will be inferior. On the other hand, the temperature over 800 °C undesirably reaches to the transition temperature of Ti. When the transition takes place in the titanium (Ti) or titanium (Ti) alloy substrate, its mechanical strength deteriorates.

### Brief Description of Drawings

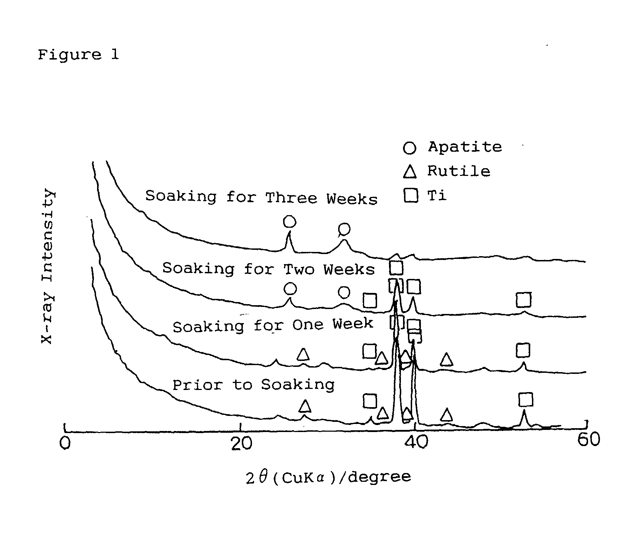

Figure 1 indicates the result of thin layer X-ray diffraction analysis performed on the test plates which were heat-treated at 600 °C after being soaked in the NaOH solution, then soaked into the simulated body fluid.

### Best Mode for Carrying Out the Invention

Titanium (Ti) metal plates of 15 X 10 X 1 mm³ size were polished with #400 diamond paste, washed with acetone and subsequently with distilled water. These plates were soaked in either 10 M-NaOH or -KOH aqueous solutions at 60 °C for 24 hours. Then these test plates were washed with distilled water using a sonic cleaner for at least 20 minutes. Their surfaces were observed to be of a uniform yellow tint and of a uniform yellow for the plates soaked in NaOH and KOH, respectively, which confirmed the formation of alkali titanates.

Then, the Ti metal plates were placed in a furnace and the temperature was raised to 400, 500, 600 and 800 °C at a heating rate of 5 °C/min, and maintained at these given temperatures for an hour. The changes in the surface structure of the titanium metal plates after the said treatments were observed by the methods of thin film X-ray diffraction and scanning electron microscope-energy dispersive X-ray analysis (SEM-EDX).

In the thin film X-ray diffraction photograph of the test plates after being soaked in alkali solutions, broad peaks caused by the amorphous phase were observed at a 2θ value between 23 and 30 °C. The formation of this phase is attributed to the reaction between titanium oxide and alkali ions. As the temperature of the plate heat treatment increases, the intensity of the titanium oxide crystalline phase peak also increased in the X-ray diffraction. When the heating temperature is at most 600 °C, there existed also the peaks of the amorphous phase. On the other hand, the heat treatment at 800 °C resulted in the disappearance of the amorphous phase peak and, instead many peaks of crystalline titanium oxide and alkali titanates appeared. On the surface of the test plate which was heat-treated at 600 °C, there was about 1 µm thickness of an amorphous layer, which covered the surface of the titanium metal uniformly. Further, the cross-section SEM-EDX observation of the test plate which was heat treated at 600 °C after soaking in KOH solution revealed that the concentration of potassium is gradually decreasing from the surface to the inside of the amorphous layer.

These results are summarize in Table 1.

**Table 1**

| No. | Alkali Solution | Heating Temp. (°C) | Constitution Phase of the Primary Surface Layer | Appearance |
|---|---|---|---|---|
| 1 | NaOH | 400 | Ti+ Amorphous TiO₂ + Amorphous alkali titanate | Uniform |
| 2 | NaOH | 500 | Ti + Rutile + Amorphous TiO₂ + Amorphous alkali titanate | Uniform |
| 3 | NaOH | 600 | Ti + Rutile + Amorphous alkali titanate | Uniform |
| 4 | NaOH | 800 | Ti + Rutile + Na₂Ti₅O₁₂ | Uniform |
| 5 | KOH | 400 | Ti + Amorphous TiO₂ + Amorphous alkali titanate | Uniform |
| 6 | KOH | 500 | Ti + Anatase + Amorphous TiO₂ + Amorphous alkali titanate | Uniform |
| 7 | KOH | 600 | Ti + Anatase + Amorphous alkali titanate | Uniform |
| 8 | KOH | 800 | Ti+ Anatase + K₂Ti₅O₁₂ + K₂TiO₃ | Uniform |

As seen in Table 1, the crystalline titanium oxide phases (a rutile type and an anatase type ) and amorphous alkali titanate phases have been formed on the surface of the Ti metal plates which were heat-treated at the temperature from 400 to 600 °C. On the other hand, the amorphous alkali titanate phase has disappeared from the Ti metal surface heated at 800 °C, and the crystalline phase of Na₂Ti₅O₁₂ was confirmed, instead.

Then, the obtained test plates were soaked into the simulated body fluid which consists of the inorganic ion concentration almost equal to human body fluid, and the formation of apatite layer was examined. The simulated body fluid was prepared by having the following ion concentrations: K⁺, 5.0; Na⁺, 142; Mg²⁺, 1.5; Ca²⁺, 2.5; Cl⁻, 148; HCO₃⁻, 4.2; HPO₄²⁻, 1.0; and SO₄²⁻, 0.5; each in mM units, and by having its pH value controlled to 7.4 at 37 °C by tri-(hydroxy methyl)-aminomethane and hydrochloric acid.

Figure 1 indicates the result of thin film X-ray diffraction analysis performed on the test plates which were heat-treated at 600 °C after being soaked in the NaOH solution, then subsequently soaked into the simulated body fluid. Within the two weeks of soaking, apatite started to grow on the surface. After three weeks, the grown apatite layer covered the surface and the titanium metal peaks were hardly observed. After soaking for three weeks, the apatite layer with a 5 to 10 µm thickness formed on the surface of the titanium metal homogeneously. The same tendency was also observed for test plates heat-treated at 400 °C and 500 °C.

### Industrial Applicability

A material for a bone substitute and its manufacturing method in the present invention consist of the said constitution, therefore they offer the following remarkable effects.

The manufacturing process involves a simple heating after soaking the Ti metal or Ti alloy into the alkali solution, thereby avoiding any expensive device. When this material is inserted in the body, the apatite, which is the same as the inorganic components in bones, forms naturally on its surface. Through the apatite, the material bonds with bones tightly. Further, when the material is soaked into the aqueous solution which comprises calcium ions and phosphorous ions at the level which exceeds the saturated concentration of apatite, or more desirably into the simulated body fluid, the apatite, which is the same as the inorganic components in bones, forms on the material's surface. Through the apatite, the material bonds with bones tightly. Consequently, this material is superior in biocompatibility.

In addition, the substrate made of Ti metal or Ti alloy and the second surface layer made of apatite bond through the primary surface layer made of titanium oxide and so on, by the means of chemical bonding and with a gradual concentration slope. Therefore, the adhesive strength of apatite towards the substrate is large.

## Claims

1. A material suitable as a bone substitute which comprises a substrate made of titanium Ti or titanium Ti alloys and a surface layer (primary surface layer) formed on the surface of the substrate, said primary surface comprising a titanium oxide phase and amorphous phases of alkali titanates.

2. The material according to claim 1, wherein the material possesses the second layer, mainly comprising apatite, further on top of the said primary surface layer.

3. The material according to claims 1 or 2, wherein the concentration of titanium oxide phase in the primary surface layer is gradually reducing towards the outer surface, while the concentration of the total alkali ions in the primary surface layer is gradually increasing towards the outer surface.

4. The material according to any of claims 1 through 3, wherein the thickness of the primary surface layer is 0.1 to 10 µm.

5. The material according to any of claims 1 through 4, wherein the thickness of the second surface layer is at least 1 µm.

6. A process for producing a material suitable as a bone substitute
, comprising the steps of:
soaking a substrate made of titanium Ti or titanium Ti alloys into alkaline solution; and
followed by heating the substrate to the level of at most the titanium Ti transition temperature.

7. A process for producing a material suitable as a bone substitute , comprising the steps of:
soaking of the substrate made of titanium Ti or titanium Ti alloys into alkaline solution;
heating the substrate to the level of at most the titanium Ti transition temperature; and
soaking in the aqueous solution which contains calcium Ca and phosphorous P to a level of, at least, the apatite solubility.

8. The process according to claims 6 or 7, wherein the said aqueous solution contains at least one of : sodium ions Na+, potassium ions K+, and calcium ions Ca2+.

9. The process according to any of claims 6 through 8, wherein the heating temperature is from 300 to 800 °C.

10. The process according to any of claims 6 through 8, wherein the heating temperature is form 550 to 650 °C.

## Patentansprüche

1. Ein als Knochenersatzstoff geeignetes Material, umfassend ein aus Titan oder Titanlegierungen hergestelltes Substrat und eine auf der Oberfläche des Substrats ausgebildete Oberflächenschicht (primäre Oberflächenschicht), wobei die primäre Oberfläche eine Titanoxid-Phase und amorphe Phasen von Alkalititanaten umfaßt.

2. Material nach Anspruch 1, worin das Material weiterhin oben auf der primären Oberflächenschicht eine zweite hauptsächlich Apatit umfassende Schicht aufweist.

3. Material nach den Ansprüchen 1 oder 2, worin die Konzentration der Titanoxid-Phase in der primären Oberflächenschicht graduell in Richtung der äußeren Oberfläche abnimmt, während die Konzentration der gesamten Alkaliionen in der primären Oberflächenschicht graduell in Richtung der äußeren Oberfläche zunimmt.

4. Material nach einem der Ansprüche 1 bis 3, worin die Dicke der primären Oberflächenschicht 0,1 bis 10 µm beträgt.

5. Material nach einem der Ansprüche 1 bis 4, worin die Dicke der zweiten Oberflächenschicht wenigstens 1 µm beträgt.

6. Verfahren zur Herstellung eines als Knochenersatzstoff geeigneten Materials, umfassend die Schritte:
Einweichen eines aus Titan oder Titanlegierungen hergestellten Substrats in alkalischer Lösung, und
nachfolgendes Erhitzen des Substrats auf höchstens den Pegel der Titan-Übergangstemperatur.

7. Verfahren zur Herstellung eines als Knochenersatzstoff geeigneten Materials, umfassend die Schritte:
Einweichen des aus Titan oder Titanlegierungen hergestellten Substrats in alkalischer Lösung;
Erhitzen des Substrats auf höchstens den Pegel der Titan-Übergangstemperatur, und
Einweichen in einer wäßrigen Lösung, welche Calcium und Phosphor mit einem Pegel von wenigstens der Apatit-löslichkeit enthält.

8. Verfahren nach den Ansprüchen 6 oder 7, worin die wäßrige Lösung wenigstens eines von: Natriumionen Na⁺, Kaliumionen K⁺ und Calciumionen Ca2⁺ enthält.

9. Verfahren nach einem der Ansprüche 6 bis 8, worin die Heiztemperatur 300 bis 800°C beträgt.

10. Verfahren nach einem der Ansprüche 6 bis 8, worin die Heiztemperatur 550 bis 650°C beträgt.

## Revendications

1. Matériau approprié comme produit de substitution des os qui comprend un substrat formé de titane Ti ou d'alliages de titane Ti et une couche superficielle (couche superficielle primaire) formée sur la surface du substrat, ladite couche primaire comprenant une phase d'oxyde de titane et des phases amorphes de titanates alcalins.

2. Matériau selon la revendication 1 où le matériau possède une seconde couche, comprenant principalement de l'apatite, au-dessus de ladite couche superficielle primaire.

3. Matériau selon la revendication 1 ou 2 où la concentration de la phase d'oxyde de titane dans la couche superficielle primaire diminue progressivement en direction de la surface externe tandis que la concentration des ions alcalins totaux dans la couche superficielle primaire augmente progressivement en direction de la surface externe.

4. Matériau selon l'une quelconque des revendications 1 à 3 où l'épaisseur de la couche superficielle primaire est de 0,1 à 10 µm.

5. Matériau selon l'une quelconque des revendications 1 à 4 où l'épaisseur de la seconde couche superficielle est d'au moins 1 µm.

6. Procédé de production d'un matériau approprié comme produit de substitution des os comprenant les étapes suivantes :
immersion d'un substrat formé de titane Ti ou d'alliages de titane Ti dans une solution alcaline, puis
chauffage du substrat au plus au niveau de la température de transition du titane Ti.

7. Procédé de production d'un matériau approprié comme produit de substitution des os comprenant les étapes suivantes :
immersion d'un substrat formé de titane Ti ou d'alliages de titane Ti dans une solution alcaline,
chauffage du substrat au plus au niveau de la température de transition du titane Ti et
immersion dans une solution aqueuse qui contient du calcium Ca et du phosphore P au moins au niveau de la solubilité de l'apatite.

8. Procédé selon la revendication 6 ou 7 où ladite solution aqueuse contient au moins un type d'ions parmi : les ions sodium Na⁺, les ions potassium K⁺ et les ions calcium Ca²⁺.

9. Procédé selon l'une quelconque des revendications 6 à 8 où la température de chauffage est de 300 à 800°C.

10. Procédé selon l'une quelconque des revendications 6 à 8 où la température de chauffage est de 550 à 650°C.
